(19) Europäisches Patentamt

European Patent Office

Office européen des brevets

(11) EP 1 727 066 A2

(12) EUROPEAN PATENT APPLICATION

(43) Date of publication:
29.11.2006 Bulletin 2006/48

(51) Int Cl.:
*G06F 19/00* (2006.01)

(21) Application number: 06251527.5

(22) Date of filing: 22.03.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 31.10.2005 US 264259
17.05.2005 US 682048 P

(71) Applicant: Agilent Technologies, Inc.
Palo Alto, CA 94306 (US)

(72) Inventors:
• Vailaya, Aditya
Loveland, CO 80537-0599 (US)

• Kuchinsky, Allan
Loveland, CO 80537-0599 (US)
• King, Jennifer Y.
Loveland, CO 80537-0599 (US)
• Ferrara, Rossella
Loveland, CO 80537-0599 (US)
• Quertermous, Thomas
Loveland, CO 80537-0599 (US)

(74) Representative: Exell, Jonathan Mark
Williams Powell
Morley House
26-30 Holborn Viaduct
London EC1A 2BP (GB)

(54) **Network-based approaches to identifying significant molecules based on high-throughput data analysis**

(57) Methods, systems and computer readable media for network-based identification of significant molecules, for which at least one biological network (100) is provided to include significant molecules to be identified. A node in the network is identified. A member-specific sub-network containing nodes connected to the identified node is identified for L levels of nearest neighbors, wherein L is a positive integer, and a connectivity score (214) is calculated for the molecule represented by the identified node based on significance scores (210, 308) of each node contained in the member-specific sub-network. These steps are repeated for other nodes in the network.

FIG. 7

EP 1 727 066 A2

## Description

### CROSS-REFERENCE

This application is a continuation-in-part application of Serial No. 10/641,492, filed August 14, 2003, pending which is incorporated herein by reference in its entirety and to which application we claim priority under 35 USC §120. This application also claims the benefit of U.S. Provisional Application No. 60/682,048, filed May 17, 2005, which application is incorporated herein, in its entirety, by reference thereto.

### BACKGROUND OF THE INVENTION

The development of microarray technology has grown from modest beginnings to the present day where the ability to expression profile whole genomes is routine. However, high throughput gene expression profiling presents a unique difficulty in the need to identify and distinguish significant changes in gene expression from among the tens of thousands of genes that can be assayed simultaneously. Indeed, analysis of high throughput data in the context of disease processes can be a daunting task. Statistical algorithms such as Significance Analysis of Microarrays (SAM) and hierarchal clustering have been developed to help facilitate analysis of gene expression data from microarrays.

The SAM algorithm assigns a score to each gene represented on a microarray on the basis of change in gene expression relative to the standard deviation of repeated measurements, see Tusher et al., "Significance analysis of microarrays applied to the ionizing radiation response", 5116-5121, PNAS, April 24, 2001, vol. 98, no. 9, which is hereby incorporated herein, in its entirety, by reference thereto. For genes with scores greater than an adjustable threshold, SAM uses permutations of the repeated measurements to estimate the percentage of genes identified by chance, the false discovery rate (FDR). However, a list of significantly regulated genes does not provide much context to the biologist studying a disease.

Hierarchical clustering applies statistical algorithms to group genes according to similarity among gene expression patterns, where similarity values are typically calculated by Euclidean distance or correlation coefficient, e.g., see Larkin et al., "Cardica transcriptional response to acute and chronic angiotensin II treatments", Physiol Genomics, 18: 152-166, 2004, which is hereby incorporated herein, in its entirety, by reference thereto. Hierarchical clustering technique do not provide context to the disease or phenomenon being studied, but are useful in identifying and distinguishing sets of statistically significant genes.

Other approaches having included conducting studies using other analytical approaches in combination with SAM statistics. In particular, an article by Lopes et al., Pathophysiology of plaque instability: insights at the genomic level", Prog Cardi ovasc Dis 44: 323-328, 2002, which is incorporated herein, in its entirety, by reference thereto, discusses the importance of identification of gene groupings towards developing an understanding of the causes and risks for atherosclerosis.

Although hierarchical clustering has been used as a pathway discovery tool (changes in expression of genes in activated networks would be expected to correlate, see Johnson et al., "Genomic profiles and predictive biological networks in oxidant-induced atherogenesis", Physiol Genomics 13: 263-275, 2003, which is incorporated herein, in its entirety, by reference thereto) this ignores, among other things, the fact that some proteins are not transcriptionally regulated.

PathwayAssist, a commercially available pathway discovery program (Ariadne Genomics, http://www.ariadnegenomics.com/products/pathway.html) may be used to develop a pathway based upon genes identified as significant by any of the techniques described above. Although this program offers functionality as a pathway discovery tool, it lacks both objectivity and any form of mathematical expression of the connectedness of the genes plotted in the pathway that it generates.

More powerful tools and approaches are needed to provide context to high throughput data as it relates to a disease or other condition being studied, and for which the experiments that generated the high throughput data were conducted.

### SUMMARY OF THE INVENTION

Methods, systems and computer readable media for network-based identification of significant molecules, for which at least one biological network is provided to include significant molecules to be identified. A node is identified in the network. A member-specific sub-network containing nodes connected to the identified node is identified for L levels of nearest neighbors, wherein L is a positive integer, and a connectivity score is calculated for the molecule represented by the identified node based on significance scores of each node contained in the member-specific sub-network. These steps are repeated for other nodes in the network.

Methods, systems and computer readable media for network-based identification of significant molecules, for which at least one biological network is provided to include significant molecules to be identified, a data set including data values characterizing molecules experimented on is provided, and an interesting list of molecules is provided as a subset of the molecules from the dataset, the interesting list including significance scores for the molecules in the list. Such identification includes identifying a node in the network; identifying a member-specific sub-network containing nodes connected to the identified node for L levels of nearest neighbors, wherein L is a positive integer; extracting the member-specific sub-network from the network; and repeating the steps of identifying a node, identifying a member-specific network and extracting the member-specific sub-network form the network for each of the other nodes in the network that corresponds to a molecule in the interesting list.

These and other advantages and features of the invention will become apparent to those persons skilled in the art upon reading the details of the methods, systems and computer readable media as more fully described below.

## BRIEF DESCRIPTION OF THE DRAWINGS

The patent or application file contains at least one drawing executed in color. Copies of this patent or patent application publication with color drawing(s) will be provided by the Office upon request and payment of the necessary fee.

Fig. 1 is a simplified illustration of a biological diagram that models interactions between a number of molecules.

Fig. 2 is an illustration of an interesting sub-network of the network of Fig. 1.

Fig. 3 is an illustration of a data sub-network extracted from the network of Fig. 1.

Fig. 4 shows a portion of a chart that was constructed for a study of diabetes in atherosclerotic patients, after calculating connectivity scores for each member.

Fig. 5 shows a portion of a chart that was generated from the same data analyzed in the example shown in Fig. 4, but using a different network to start with.

Fig. 6 shows a super-network that was generated from the member-specific sub-networks extracted for those members on the interesting list in the experiment described with regard to Fig. 5.

Fig. 7 illustrates a typical computer system in accordance with an embodiment of the present invention.

## DETAILED DESCRIPTION OF THE INVENTION

Before the present methods, systems and computer readable media are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a sub-network" includes a plurality of such sub-networks and reference to "the node" includes reference to one or more nodes and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## DEFINITIONS

An "interesting list" refers to a list of molecules for a disease process and/or condition under study associated with some high throughput data that have been determined to be significantly differentially regulated relative to other molecules represented in a population of high throughput data, which may be high throughput data from gene expression, location analysis, proteomic and/or metabolomic studies.

"Nexus genes" refer to potentially regulatory molecules that may or may not be members of an "interesting list" of molecules and that are associated with a number of other molecules (at least some of which are members of the interesting list) in a biological diagram or biological network.

The term "local format" or "local formatting" refers to a common format into which knowledge extracted from textual documents, biological data and biological diagrams can all be converted so that the knowledge can be interchangeably used in any and all of the types of sources mentioned. The local format may be a computing language, grammar or Boolean representation of the information which can capture the ways in which the information in the three categories are represented. The local format thus refers to a restricted grammar/language used to represent extracted semantic information from diagrams, text, experimental data, etc., so that all of the extracted information is in the same format and may be easily exchanged and used in together. The local format can be used to link information from diverse categories, and this may be carried out automatically. The information that results in the local format can then be used as a precursor for application tools provided to compare experimental data with existing textual data and biological models, as well as with any textual data or biological models that the user may supply, for example.

The term "biological diagram", "biological model" or "pathway", as used herein, refers to any graphical image, stored in ay type of format (e.g., GIF, JPG, TIFF, BMP, etc.) which contains depictions of concepts found in biology. Biological diagrams include, but are not limited to, pathway diagrams, cellular networks, signal transduction pathways, regulatory pathways, metabolic pathways, protein-protein interactions, interactions between molecules, compounds, or drugs, and the like. A "biological network" refers to a graph representation (which may also include text, and other information) wherein biological entities and the interrelationships between them are represented as diagrammatic nodes and links, respectively. Examples of biological networks include, but are not limited to pathways and protein-protein interaction maps. A "pathway" refers to an ordered sequence of interactions in a biological network. An example of a pathway is a cascade of signaling events, such as the wnt/betacatenin pathway, which represents the ordered sequence of interactions in a cell as a result of an outside stimulus, in this case, the binding of the wnt ligand to a receptor on the membrane of the cell. The terms "pathway" and "biological network" are sometimes used interchangeably in the art.

A "biological concept" or "concept" refers to any concept from the biological domain that can be described using one or more nouns according to the techniques described herein.

A "relationship" or "relation" refers to any concept which can link or "relate" at least two biological concepts together. A relationship may include multiple nouns and verbs.

An "entity" or "item" is defined herein as a subject of interest that a researcher is endeavoring to learn more about, and may also be referred to as a biological concept, i.e., "entities" are a subset of "concepts". For example, an entity or item may be one or more genes, proteins, molecules, ligands, diseases, drugs or other compounds, textual or other semantic description of the foregoing, or combinations of any or all of the foregoing, but is not limited to these specific examples.

An "interaction" as used herein, refers to some association relating two or more entities. Co-occurrence of entities in an interaction implies that there exists some relationship between those entities. Entities may play a number of roles within an interaction. The structure of roles in an interaction determines the nature of the relationship(s) amongst the various entities that fill those roles. Interactions may be considered a subset of relationships.

A "node" refers to an entity, which also may be referred to as a "noun" (in a local format, for example). Thus, when data is converted to a local format nodes are selected as the "nouns" for the local format to build a grammar, language or Boolean logic.

A "link" refers to a relationship or action that occurs between entities or nodes (nouns) and may also be referred to as a "verb" (in a local format, for example). Verbs are identified for use in the local format to construct a grammar, language or Boolean logic. Examples of verbs, but not limited to these, include up-regulation, down-regulation, inhibition, promotion, bind, cleave and status of genes, protein-protein interactions, drug actions and reactions, etc.

"Phosphorylation" refers to the addition of phosphate groups to hydroxyl groups on proteins (side chains s, T or Y) catalysed by a protein kinase often specific) with ATP as phosphate donor. Activity of proteins is often regulated by phosphorylation. Phosphorylation is one type of post-translational protein modification mechanism.

"Activated" refers to the state of a biochemical entity wherein it is enabled for performing its function.

"Inhibited" is used to refer to the state of a biochemical entity wherein it is wholly or partially disabled or deactivated for performing its function.

"Up-regulated" refers to a state of a gene wherein its production of corresponding RNA (ribonucleic acid) transcript is significantly higher than in a reference condition.

"Down-regulated" refers to refers to a state of a gene wherein its production of corresponding RNA transcript is significantly lower than in a reference condition.

A "co-factor" is an inorganic ion or another enzyme that is required for an enzyme's activity.

A "rule" refers to a procedure that can be run using data related to stencils, nodes, and links. Rules can be declarative assertions that can be computationally verified, for example "an enzyme must be a protein", or they can be arbitrary procedures that can be computationally executed using data related to stencils, nodes, and links, for example "if there is a relation such that entity A activates entity B, and if A is in state activated, then set B in state activated".

A "stencil" refers to a diagrammatic representation which may contain one or more biological concepts, entities, times, interactions, relationships and descriptions (generally, although not necessarily, graphic descriptions) of how these interact. Stencils function similarly to macros in Microsoft Word or Excel, with respect to their functionality for generating more than one node or link at a time when constructing a biological diagram. Stencils may be comprised of graphical elements, such as shapes (e.g. rectangles, ovals), lines, arcs, arrows, and/or text. These elements have biological semantics; that is, elements represent types of biological entities, such as genes, proteins, RNA, metabolites, compounds, drugs, complexes, cell, tissue, organisms, biological relationship, disease, or the like.

A "database" refers to a collection of data arranged for ease and speed of search and retrieval. This term refers to an electronic database system (such as an Oracle database) that would typically be described in computer science literature. Further this term refers to other sources of biological knowledge including textual documents, biological diagrams, experimental results, handwritten notes or drawings, or a collection of these.

A "biopolymer" is a polymer of one or more types of repeating units. Biopolymers are typically found in biological systems and particularly include polysaccharides (such as carbohydrates), and peptides (which term is used to include polypeptides and proteins) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another.

A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.. For example, a "biopolymer" includes DNA (including cDNA), RNA, oligonucleotides, and PNA (peptide nucleic acid) and other polynucleotides, regardless of the source. An "oligonucleotide" generally refers to a nucleotide multimer of about 10 to 100 nucleotides in length, while a "polynucleotide" includes a nucleotide multimer having any number of nucleotides. A "biomonomer" references a single unit, which can be linked with the same or other biomonomers to form a biopolymer (for example, a single amino acid or nucleotide with two linking groups one or both of which may have removable protecting groups).

A "chemical array", "array" or "microarray", unless a contrary intention appears, includes any one-, two- or three-dimensional arrangement of addressable regions bearing a particular chemical moiety or moieties (for example, biopolymers such as polynucleotide sequences) associated with that region. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other). An "array layout" refers to one or more characteristics of the features, such as feature positioning on the substrate, one or more feature dimensions, and an indication of a moiety at a given location. "Hybridizing" and "binding", with respect to polynucleotides, are used interchangeably. A "pulse jet" is a device which can dispense drops in the formation of an array. Pulse jets operate by delivering a pulse of pressure to liquid adjacent an outlet or orifice such that a drop will be dispensed therefrom (for example, by a piezoelectric or thermoelectric element positioned in a same chamber as the orifice).

When one item is indicated as being "remote" from another, this is referenced that the two items are at least in different buildings, and may be at least one mile, ten miles, or at least one hundred miles apart.

"Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

A "processor" references any hardware and/or software combination which will perform the functions required of it. For example, any processor herein may be a programmable digital microprocessor such as available in the form of a mainframe, server, or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic or optical disk may carry the programming, and can be read by a suitable disk reader communicating with each processor at its corresponding station.

"May" means optionally.

Methods recited herein may be carried out in any order of the recited events which is logically possible, as well as the recited order of events.

A pathways-based approach to analysis of high throughput data as described herein may provide context for identifying significant therapeutics from among a large list of significantly regulated genes or other high throughput data determined to be significantly differentiated from a larger total population of that high throughput data. Identification of networks of interactions between significant genes represented by the significant high throughput data, may provide crucial information for complex diseases where multiple genes and the environment interact. Described herein are methods and systems-based approaches to studying complex diseases in terms of gene-gene interactions among significantly regulated genes. Further, highly connected genes may be identified, referred to as 'nexus' genes, which may be considered attractive candidates for therapeutic targeting.

A pathways-based approach can account for the fact that some proteins are not transcriptionally regulated and, at the same time, take account of prior knowledge by expanding the context beyond the genes and gene changes in the current experiment. A more comprehensive analysis of this type is particularly suited for complex diseases, where genes and the environment interact. It is not realistic, for example, to attempt to understand the inner workings of an automobile simply by disassembling it into its various parts, determining vital components and choosing to study those individual components. In the same way, analysis of a complex disease should be conducted with a more systems-based approach that allows for in-depth study of gene-gene interactions, and gives prominence to interactions among genes known to be differentially modulated in disease progression.

Described are systems, methods and computer readable media for analyzing disease processes in terms of gene-gene interactions and/or for identification of highly connected genes as potential therapeutic targets. Input for these analysis techniques may be high throughput data from gene expression, genotyping, location analysis, proteomic and/or metabolomic studies from which significantly differentially regulated molecules for the disease process have been identified. A list of such significantly differentially regulated molecules is referred to herein as an "interesting list".

Each of the molecules in the interesting list has a score associated with it, which represents its significance. The score is referred to as the "significance score". For example, the score can be based on the d-scores associated with SAM analysis or the ranking of molecules in terms of the relative differential expression (most differentially expressed molecules have lower ranks, hence higher scores). A comprehensive network of molecular interactions involving molecules in the interesting list may be constructed from any one or combination of the following: (i) language parsing of published literature; (ii) merging of existing pathway databases, metabolic reaction databases, protein-protein interaction databases; (iii) manually created network maps; and (iv) automatic network generation from experimental data.

A method and system for knowledge extraction is described in co-pending, commonly owned Application Serial No. 10/154,524 titled "System and Method for Extracting Pre-Existing Data from Multiple Formats and Representing Data in a Common Format for Making Overlays", filed on May 22, 2002. Application Serial No. 10/154,524 is hereby incorporated by reference herein, in its entirety, by reference thereto. Further, a method and system for using local user context to extract relevant knowledge is described in co-pending and commonly assigned Application Serial No. 10/155,304 filed May 22, 2002 and titled "System, Tools and Methods to Facilitate Identification and Organization of New Information Based on Context of User's Existing Information". Application Serial No. 10/155,304 is hereby incorporated by reference herein, in its entirety, by reference thereto. Described are methods and systems wherein automated text mining techniques are used to extract "nouns" (e.g. biological entities) and "verbs" (e.g. relationships) from sentences in scientific text. Thus, knowledge extraction from scientific literature, e.g. via text mining, can identify biological entities that are involved in a relationship, for example a promotion interaction involving two genes. The resulting interpretation is represented in a restricted grammar, referred to as "local format".

Co-pending and commonly owned Application Serial No. 10/642,376 filed August 14, 2003 and titled "System, Tools and Method for Viewing Textual Documents, Extracting Knowledge Therefrom and Converting the Knowledge into Other Forms of Representation of the Knowledge" describes conversion of text to the local format using an interactive text viewing tool. This tool can automatically identify and extract entities and relationships found in a passage of text, and then provide an interface by which a user can interactively refine and disambiguate the extracted knowledge, which the present invention converts to a local format, thereby greatly improving the accuracy and reliability of the knowledge generated, as a result of the process. The local format serves as a structured way for the user to review and encode the relevant knowledge contained in scientific text. It also serves as a biological object model that can be manipulated by other computational tools. Application Serial No. 10/642,376 is hereby incorporated by reference herein, in its entirety, by reference thereto.

Co-pending and commonly assigned Application Serial No. 10/641,492 filed August 14, 2003 and titled "Method and System for Importing, Creating and/or Manipulating Biological Diagrams" discloses systems and methods for mapping biological concepts and relationships to regions, on graphical images that have biological semantic meaning, where those concepts and relationships are located. Such superimposition allows researchers to examine their data of interest in the form that they prefer (e.g., native data format, text format or graphical format) in the context of previously defined knowledge which is represented by the diagram. Moreover, such an overlay can allow for easy understanding of data with respect to a static model represented by the diagram.

Biological diagrams may be generated from a variety of input formats. The system may import graph data structures from pre-existing databases, for example. Separate import modules may serve on a database-specific basis to allow a biological diagram to be created given information in the format of each such specific database. A collection of local format objects may be imported to the system to construct a biological diagram. Diagrams created and/or imported by the present system may be saved and loaded.

Another functionality provided is the ability to import static graphical images and convert them to interactive biological diagrams. For example, a system may process an image of a biological diagram and determine a mapping to the coordinates of biological concepts found in the graphic. As noted above, the system can process diagrams from virtually any source. Examples of such sources include, but are by no means limited to: Boehringer-Mannheim charts, Kyoto Encyclopedia of Genes and Genomes (KEGG), and directed acyclic graphs of the Gene Ontology (GO) classification scheme. The system may also simultaneously make use of a combination of diagrams from a single source or a combination of sources. Further details and capabilities of the above-described systems and methods are found in Application Serial No. 10/641,492, which is hereby incorporated herein, in its entirety, by reference thereto.

Co-pending, commonly assigned Application Serial No. 10/784,523 filed on February 23, 2004 and titled "System, Tools and Method for Constructing Interactive Biological Diagrams" provides a visual grammar, to accompany the local format, and to represent interrelationships amongst biological entities and activities. The visual grammar is based upon a library of stencils that graphically represent common types of biological entities and connections between them. The present invention also provides lightweight software tools for composing and editing the stencils, as well as tools for linking the elements of stencils, and their values, to other data elements, datasets, and the local format. Stencils may be comprised of graphical elements, such as shapes (e.g. rectangles, ovals), lines, arcs, arrows, and text. These elements have biological semantics; that is, elements represent types of biological entities, such as genes, proteins, RNA, metabolites, compounds, drugs, complexes, cell, tissue, organisms, biological relationship, disease, or the like.

The biological semantics facilitate linking of the stencils with other forms of biological data. Further, stencils represent composites of biological activity, and therefore may function like "macros" for easier and more rapid building of biological diagrams. Stencils permit two-way interactions between textual documents and diagrams, or between diagrams and other forms of data such as experimental data, for example. Further stencils support user-controlled graphical exploration of alternatives, such as alternatives to pre-existing diagrams. Stencils may be used collaboratively among multiple users, whether by providing a blank set of stencils as a starter template, sharing of filled-in stencils, collaboratively filling in stencils, or any combination of these. Further details about stencils and systems for building biological diagrams are found in Application Serial No. 10/784,523, which is hereby incorporated herein, in its entirety, by reference thereto.

Based upon at least one dataset produced by a gene expression, genotyping, location analysis, proteomic or metabolomic study (the invention is particularly well-suited to datasets produced by high throughput techniques) and an interesting list of members of the at least one dataset that have been determined to be differentiated from the remainder of the population of the dataset(s), in addition to a biological diagram that models interactions between the members included in the dataset(s), the present invention further processes this information to provide more contextual meaning of the data as it relates to a disease or other subject of the study being conducted. As noted, the biological diagram may be a pre-existing diagram that models interactions between the members (or concepts) in the data, or may be constructed from any one or combination of the following: (i) language parsing of published literature; (ii) merging of existing pathway databases, metabolic reaction databases, protein-protein interaction databases; (iii) manually created network maps; (iv) automatic network generation from experimental data; (v) and modification of a pre-existing diagram using any of the previously mentioned sources.

A sub-network may be extracted from the biological diagram mentioned above, such that all the nodes are members of the "interesting list", which forms what is referred to as an "interesting sub-network". Another method may extract a sub-network such that all nodes of the sub-network are part of the microarray (or the given high throughput experimental data set). Such a network is referred to as a "data sub-network".

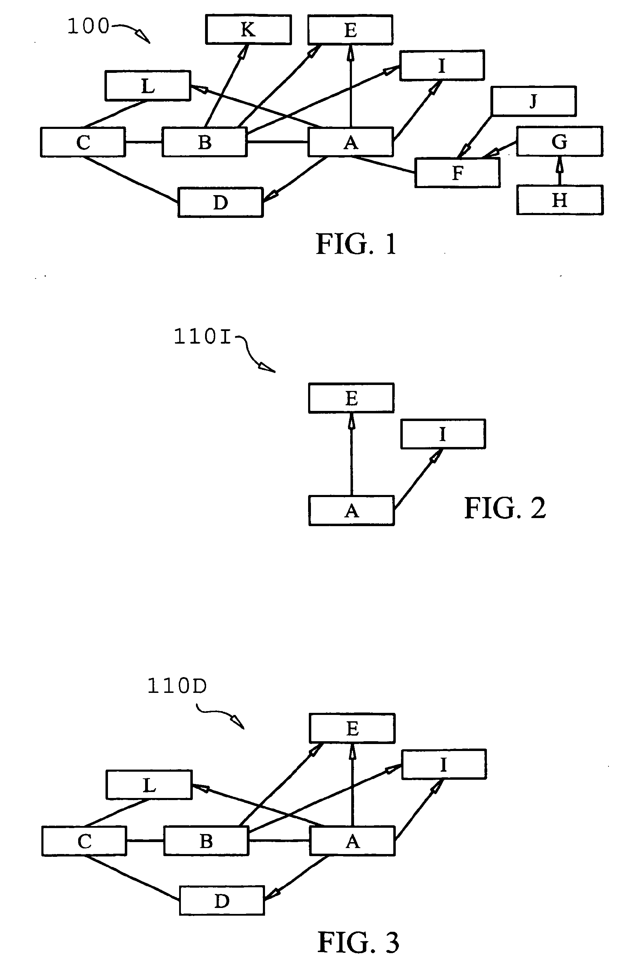

Fig. 1 is a simplified illustration of a biological diagram 100 that models interactions between a number of molecules for purposes of describing extraction of an interesting sub-network and/or a data sub-network, as referred to above. It should be noted here that diagram 100 is greatly simplified for ease of illustration and explanation, and that it is not uncommon for such diagrams to contain hundreds or thousands of nodes with interacting links, when modeling a high throughput dataset. For purposes of discussion, assume that A,B,C,D,E,I and L are members present in the data, and that A,C,E and I are also members of the interesting list. Thus, although B, D and L are in the data set and also represented on diagram 100, they are not, at this time considered to be significant and therefore are not members of the interesting list. Further, F,G,H,J and K are not members of the data, but are only members of the biological diagram being used. An extraction may be performed to identify only the interesting sub-network, which will result in interesting sub-network 110I as shown in Fig. 2. If an extraction is performed for a data sub-network, then the data sub-network 110D shown in Fig. 3 results.

Based on any of the entire network 100, the interesting sub-network 110I or the data sub-network 110D, as described above, a connectivity analysis may next be performed to rank members of the network according to connectivity scores. Note that there may be multiple disconnected sub-graphs in an extracted interesting sub-network 110I or data sub-network 110D. Further, the original biological diagram/network 100 may have multiple disconnected subdiagrams/networks. Neither of these situations impact the processing described herein, however. Whichever network is used as a basis for performing connectivity scores, each node in that network is assigned a significance score for use in computing connectivity scores.

All nodes of the interesting sub-network 110I already have assigned significance scores, as provided in the interesting list. For example, SAM or some other known statistical algorithm may be used to calculate the significance scores. When using SAM, one or two threshold values may be set for calculating the significance scores. For example, a single threshold may be set, above which, data members having significance scores having absolute values that exceed the threshold value are assigned to the interesting list. Members having significance scores, the absolute values of which do not exceed the threshold are simply assigned a significance score of zero in this case. Similarly, two threshold values may be set, a positive threshold value and a negative threshold value, the absolute values of which do not have to be equal. In this case, those members with negative significance scores need to have a significance score less than the negative threshold value to make the interesting list, and those members with positive significance scores need to have a significance score greater than the positive threshold value to make the interesting list, All other members are assigned significance scores of zero.

Alternatively, significance scores may be calculated for all members of the data set, rather than assigning significance scores of zero to those members not on the interesting list. When using the full biological diagram 100, those nodes that are not members of the dataset are assigned a significance score of zero regardless of the method used to assign significance scores to members of the dataset.

A connectivity score is computed for each member in the network 100, interesting sub-network 110I or data sub-network 110D based on identifying the links that its representative node has in the network or sub-network and by identifying the members that the node under examination links to. For example, for each member of the network 100, interesting sub-network 110I or data sub-network 110D, all its neighbors up to a pre-defined and user modifiable distance level may be extracted. Neighbors may be limited to direct interactions with other members in the network 100, interesting sub-network 110I or data sub-network 110D, or may also include indirect interactions, and this is determined by the user-modifiable distance level at the time of the connectivity score computation. Any node directly interacting with the node being currently examined/analyzed is its first neighbor. A member that is a first neighbor of the first neighbor of the node being currently analyzed, but not the first neighbor of the node being currently analyzed is the second neighbor of the node being currently analyzed, (distance = 2), and so on.

For the current node being analyzed, e.g., node A in Fig. 3, a sub-network is extracted based on the neighborhood criterion that has been set. The sub-network consisting of the node being analyzed and its neighbors is referred to as a "member-specific sub-network". A connectivity score is then computed for the member being analyzed by weighted addition of the significance scores of all its neighbors, which are provided with the members of the dataset, where weighting is based on a monotonically decreasing or non-increasing function of the distance of a given neighbor to the node being analyzed.

There are well-defined and currently available functions that may be applied to accomplish weighting, including, but not limited to: inverse of distance, exponential, etc. For weighting by inverse of distance, the weighting factor for node "i", referred to as "W(i)" is given by: W(i)=l/distance(i,A), wherein distance (i,A) is the distance of node i from node A, when node A is the node for which a connectivity score is being computed. In this case, node A is assigned a weighting value of one (i.e., W(A)=1) as the inverse distance is not defined for node A, since the distance of A from A is zero. Exponential weighting values may be calculated by Wexp(i)=exp(-distance(i,A)), values of which, like the previously mentioned calculations, decrease with increasing distance. Thus, the weighting value applied to A itself using this approach is also 1, i.e., $Wexp(A) = e^0 = 1$. Regardless of which weighting formula is applied, each resulting connectivity score may be normalized by dividing it by the sum of all the weights of the nodes considered for calculation of that connectivity score. For example, a connectivity score for node A may be defined as:

$$CS(A) = \sum_{i=1}^{n} (W_i \left| significance\, score\, (i) \right| / \sum_{i=1}^{n} W_i \qquad (1)$$

where the variable "i" represents the nodes in the neighborhood considered for calculation of the connectivity score, and "n" is the total number of nodes considered. As noted earlier, the neighborhood may be defined to include only direct interactions (first neighbors) or indirect interactions (e.g., up to and including second neighbors, where L=2, up to and including third neighbors when L=3, etc.) Note that node A is always considered to be a neighbor of node A, regardless of the value of L.

After calculating a connectivity score for each member of the network 100, interesting sub-network 110I or data sub-network 110D, the members may then be ranked (e.g., in decreasing order) according to their connectivity scores. Members with high connectivity scores are then identified as "nexus" members or highly interacting nodes representing molecules that may be potential therapeutic targets for a disease process under study.

A further normalization or thresholding function may be applied to normalize the connectivity scores of all the molecules in network 100, interesting sub-network 110I or data sub-network 110D. Some example techniques for normalization or thresholding may include (any combination of and not restricted to) the following: (i) normalize each connectivity score by dividing by the number of nodes or edges/links in the member-specific sub-network; (ii) set a threshold on the number of nodes or edges in the member-specific sub-network, such that all nodes with a corresponding sub-network with the number of nodes or edges less than the threshold are given a connectivity score of zero.

For example, the connectivity score for "A" in Fig. 3 is computed as based on the following. The significance score for "node i" is represented by SS(i). In this examples, the significance scores of the nodes are equivalent to the d-scores calculated for the same using SAM, where significance scores for nodes not on the interesting list were assigned as values of zero. Thus, only nodes A,C,E and I had non-zero significance scores, as being members of the interesting list. In this example, nodes up to and including first neighbors are considered and no weighting function, normalization or thresholding is used. Thus, the connectivity score for node A, given these constraints, is calculated by CS(A)=|SS(A)| + |SS(B)| + |SS(D)| + |SS(E)| + |SS(I)| + |SS(L)|, where |SS(i)| designates the absolute value of the significance value of node i, and where nodes B,D,E,I and L are first neighbors of node A (node A is also included in its own neighborhood). Given the same constraints, the connectivity score for node B is given by CS(B)= |SS(B)| + |SS(A)| + |SS(C)| + |SS(E)| + |SS(I)|. By normalizing the connectivity scores CS(A) and CS(B) in a manner as discussed above, using the number of nodes in each neighborhood in this example, the normalized scores are equal to CS(A)/6 and CD(B)/5, respectively. Thus, even though node B is not a member of the interesting list, it is possible for its connectivity score to be higher than that of node A, which is a member of the interesting list.

Connectivity scores may be computed directly from the biological diagram, interesting sub-network or data sub-network, without extracting member-specific sub-networks, if desired. That is, given a node, all the node's neighbors (up to the pre-defined level L) may be located by traversing the links in the network (e.g., biological network, interesting sub-network or data sub-network) and computing the connectivity score from the significance scores of the given node and identified neighboring nodes. Once accomplished, member-specific sub-networks may then be extracted to construct a super-network, as described, or a super-network extraction may be performed to extract all of the identified nodes and neighbors (or a subset thereof as determined by ranked connectivity scores that exceed a threshold) to thereby construct the super-network. Member-specific sub-networks can be determined directly from the biological diagram in the same manner as described with regard to the interesting sub-network or data sub-network. Filtering may first be performed to eliminate connectivity scores based on all nodes that have been determined to be non-significant by the fact that they do not appear on the interesting list. Alternatively, connectivity scores for all nodes may be computed.

After extracting member-specific sub-networks as described, extracted member-specific networks may be combined to form a super-network. For example, the member-specific sub-networks for the highest ranked nodes representative of the highest ranked members (those with connectivity scores greater than a user-defined and modifiable threshold) may be combined together to form a super-network of interest that potentially significantly discriminates the disease process from the normal process, or more generally, that discriminates the experimental condition being studied from the control. In other words, the super-network is constructed by merging the "member-specific sub-network" for every member whose connectivity score is greater than a threshold. If a member-specific sub-network does not have a node in common with the super-network that is being generated, it may be displayed alongside the super-network without any connecting links between it and the super-network constructed thus far. "Nexus" members refer to those members with the highest relative connectivity scores and are included within the super-network. The resulting super-network and "nexus" members define a significant context around the disease process/condition being studied, and can be further analyzed for therapeutic targeting.

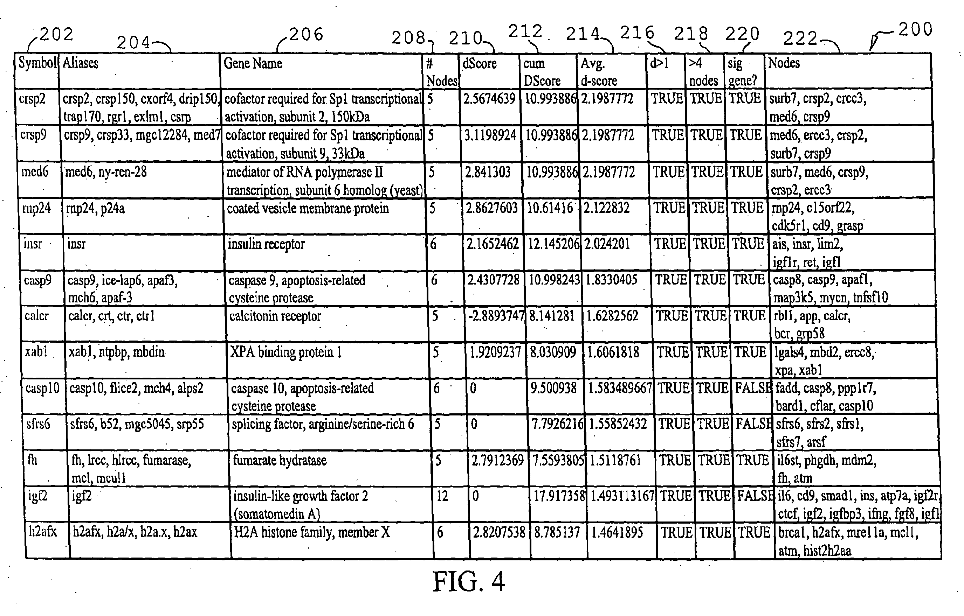

Fig. 4 shows a portion of a chart that was constructed for a study of diabetes in atherosclerotic patients, after calculating connectivity scores for each member (in this case genes represented by probes on a microarray) appearing on a biological diagram, according to the procedures described above. Significance scores for the members were calculated using SAM. It is noted that Fig. 45 shows only a very small percentage of the total number of members processed, for simplicity of illustration and discussion, as there were somewhere on the order of 22,000 genes represented in this microarray study. A data sub-network consisting of only those genes that were in the microarray data was extracted from a biological network including about 5,200 genes. Connectivity scores were then computed for each gene in the data sub-network. For each gene listed by its gene symbol under the column 202 titled "Symbol" a member-specific sub-graph (i.e., member-specific sub-network) was identified and connectivity scoring was performed for each. The column 204 labeled "Aliases" lists all other know names/symbols that the gene represented by entry in the Symbol column 202 is known to be represented by. These aliases may be determined using a biological naming system, such as described in co-pending, commonly assigned Application Serial No. 10/154,529 filed May 22, 2002 and titled "Biotechnology Information Naming System", for example. Application Serial No. 10/154,529 is hereby incorporated herein, in its entirety, by reference thereto.

The "Gene Name" column lists the name of the gene as commonly identified and may also list known or suspected functions of the gene. In column 208, the number of nodes that were identified in the member-specific sub-network for the gene reported in column 202 is reported. The significance value for the member is reported in column 210. In this case, the significance value is in terms of a d-score of the gene being reported on, as determined by SAM analysis. The significance score may be either a positive or a negative value. The higher the absolute value of the significance score, the more significant is the gene considered to be. A cumulative significance score (in this example, cumulative d-score) is calculated by summing the absolute values of the significance scores of all nodes in the member-specific sub-network and is reported in column 212. The average significance score (in this case, the average d-score) is calculated by dividing the cumulative significance score by the total number of nodes in the member-specific sub-network and is reported in column 214. Note that the connectivity score for a gene is set to the value of the average significance score calculated from the member-specific sub-network for that gene.

Columns 216 and 218 report values for thresholds that may be changed by a user. In column 216, Boolean flags (such a "0" and "1" or, as shown in Fig. 4, "TRUE" and "FALSE" are used to indicate whether the connectivity score for the member being reported on in that row surpasses the threshold that has been set. In this example, the threshold was set, such that a gene having an average significance score (connectivity score) with a value greater than one was considered to be significant.

Even if connectivity scores such as average connectivity scores are normalized, the user may wish to further filter the connectivity scores by number of nodes or number of edges/links that are contained in the member-specific sub-network being considered. Consider, for example, a case where a member-specific sub-network has only two nodes and both nodes score relatively high for significance. Even with normalizing, this member-specific sub-network will receive a high average significance score. However, another member-specific sub-network may have ten nodes with five of the nodes scoring relatively high for significance. This larger member-specific sub-network will score a substantially lower average significance score when the cumulative score is divided by ten, but may relay more useful information to a user than the member-specific sub-network containing only two nodes, since the larger member-specific sub-network contains five significant nodes/genes, while the smaller member-specific sub-network contains only two significant nodes/genes. To address this issue, the user may set a threshold so that very small member-specific sub-networks are not considered in the analysis. In the example of Fig. 4, the user has chosen to ignore member-specific sub-networks having a total of four nodes or less. As noted before, the value of this threshold may be changed by the user. As with column 216, Boolean values are entered into column 218 to indicate whether each member-specific sub-network considered passes the minimum node or link threshold requirement.

Since all genes (i.e., not only genes on the interesting list) were considered in this example, column 220 contains Boolean values to indicate whether the particular gene being considered was determined to be a significant member as determined by its significance score. The threshold level for what is considered to be significant may also be changed, as is compared to the absolute value of the significance score of the member being considered. Thus, column 220 identifies those members that make up an interesting list. Column 222 identifies the names of all nodes (representing members, in this case genes) that are included in the member-specific sub-network being considered.

Fig. 4 shows the top ranked members after sorting the chart 200 by the average significance score (Average d-score) 214. It can be observed that not all of the genes shown have surpassed all of the thresholds that were set in columns 216, 218 and 220. Thus, members that score a "FALSE" or zero (or other Boolean indicator indicating that the threshold was not surpassed) may be excluded from use in building a super-network in a manner as described above.

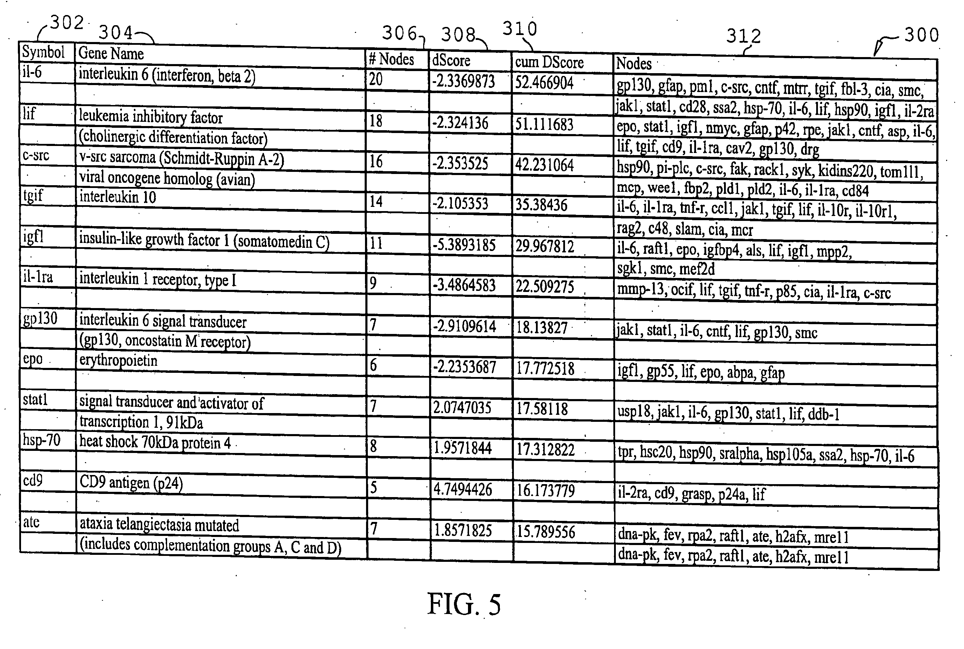

Fig. 5 shows a portion of a chart 300 that was generated from the same data analyzed in the example as shown in Fig. 4, but using a different network to start with. That is, in this example, only the interesting sub-network was used, that is, a sub-network constructed from the biological diagram to have only genes that were on the interesting list, and thus having been determined to have significant scores, was analyzed. Accordingly, Boolean indicators are not used with regard to significance of the members in chart 300, since all genes have already been determined to be significant. Member-specific sub-networks were identified in the interesting sub-diagram and the member-specific sub-networks were then analyzed to generate the information shown in Fig. 5.

Again, only a small portion of the total number of genes analyzed is shown in Fig. 5, for simplifying the explanation and illustration. Columns 302, 304 and 306 contain the same types of information as columns 202, 204 and 206, described above. Column 308 indicates the significance score of the member being reported on, in this case, the d-score of the gene as calculated by SAM. A cumulative significance score (cumulative d-score) is reported for the member-specific sub-network, as calculated by summing the absolute values of the significance scores of all nodes in the member-specific sub-network. Note that in this example, the connectivity score for a gene was set to the cumulative significance score (and hence no normalization was performed). Column 312 contains the same type of information as described above with regard to column 222.

The members in chart 300 have been sorted according to cumulative significance score (i.e., connectivity score) and may be selected for building a super-network based on this order.

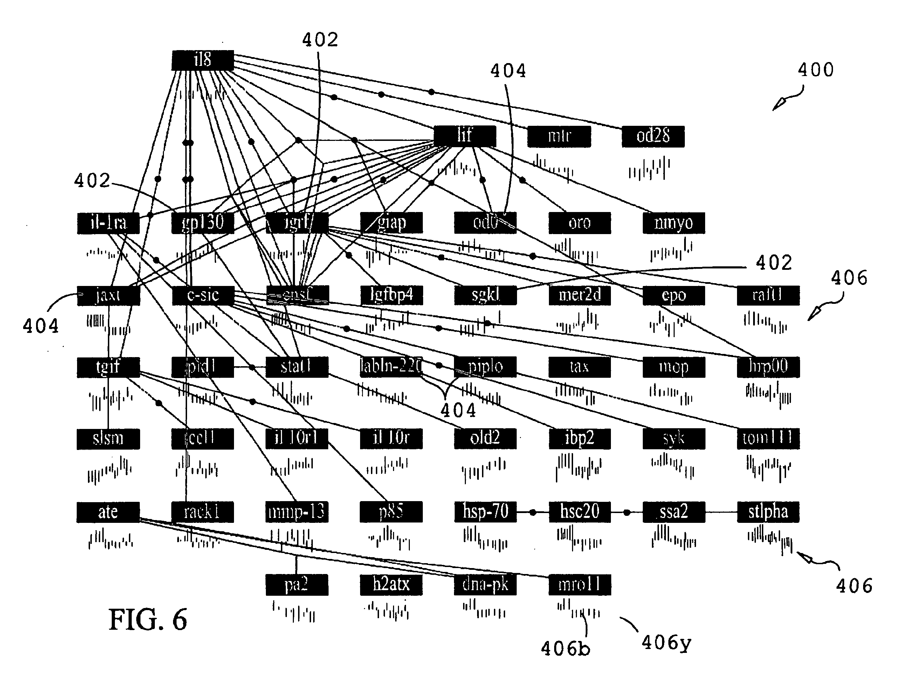

A super-network 400 was generated from the member-specific sub-networks extracted for those members on the interesting list in the experiment described with regard to Fig. 5 above (as shown in Fig. 6) which had a connectivity score (in this example, cumulative significance score) greater than a preset threshold. Bright red nodes 402 indicate genes found to be up-regulated in diabetics and relatively down-regulated or neutral in non-diabetics, while bright green nodes 404 indicate genes that are down-regulated in diabetics and relatively up-regulated or neutral in non-diabetics. In other words, the SAM d-scores are overlaid as the red (positive d-scores) and green (negative d-scores) colors on the nodes. The brighter the color, the higher is the absolute value of the significance score (d-score) for the gene. Thus, darker red nodes are up-regulated but less so than the bright red nodes. Darker green nodes are down-regulated, but less so than the bright green nodes. Black nodes are substantially neutral, i.e., not differentially regulated. Thus, shades of color coding provide a continuum of the degree to which a node is up-regulated (red) or down-regulated (green). Although the colors red, green and black are used, and intermediate shades thereof, color coding is not limited to these colors but could be any combination which is readily visually distinguishable by a user.

A heatstrip 406 is displayed beneath each node to indicate the expression level of each cell (experiment) in the row of the array for the particular gene represented by that particular node. Further details regarding the visualization of heat strips can be found in co-pending, commonly owned Application Serial No. 10/928,494 filed August 27, 2004 and titled "System and Methods for Visualizing and Manipulating Multiple Data Values with Graphical Views of Biological Relationships", which is hereby incorporated herein, in its entirety, by reference thereto. Heatstrip 406 is also color coded, where yellow bars 406y represent expression of the diabetes class and blue bars 406b represent expression of the control class (no diabetes). It can be observed from super-network 400 that nodes il6, lif, c-src, tgif, igfl and illra were the most highly connected nodes (genes) in the super-network 400, with il6 having the highest connectivity score of all (as already noted, the cumulative significance scores were used as the connectivity scores for the nodes in this experiment), having a score of 52.4669. Thus, i16 was identified as a nexus gene in coronary atherosclerosis and a key target in the pathology of diabetic coronary disease.

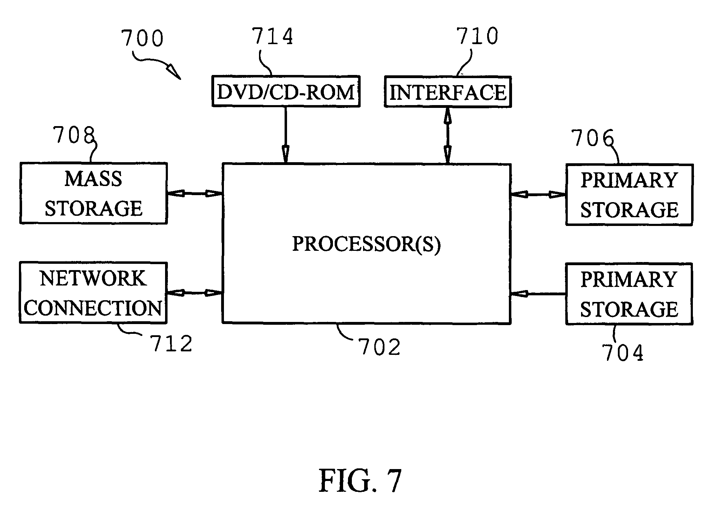

Fig. 7 illustrates a typical computer system in accordance with an embodiment of the present invention. The computer system 700 may include any number of processors 702 (also referred to as central processing units, or CPU$_S$) that are coupled to storage devices including primary storage 706 (typically a random access memory, or RAM), primary storage 704 (typically a read only memory, or ROM). As is well known in the art, primary storage 704 acts to transfer data and instructions uni-directionally to the CPU and primary storage 706 is used typically to transfer data and instructions in a bi-directional manner Both of these primary storage devices may include any suitable computer-readable media such as those described above. A mass storage device 708 is also coupled bi-directionally to CPU 702 and provides additional data storage capacity and may include any of the computer-readable media described above. Mass storage device 708 may be used to store programs, data and the like and is typically a secondary storage medium such as a hard disk that is slower than primary storage. It will be appreciated that the information retained within the mass storage device 708, may, in appropriate cases, be incorporated in standard fashion as part of primary storage 706 as virtual memory. A specific mass storage device such as a CD-ROM or DVD-ROM 714 may also pass data uni-directionally to the CPU.

CPU 702 is also coupled to an interface 710 that includes one or more input/output devices such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 702 optionally may be coupled to a computer or telecommunications network using a network connection as shown generally at 712. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for calculating connectivity scores may be stored on mass storage device 708 or 714 and executed on CPU 708 in conjunction with primary memory 706.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

**Claims**

1. A network-based method of identifying significant molecules, for which at least one biological network (100) is provided to include significant molecules to be identified, said method comprising the steps of:

   identifying a node in the network;
   identifying a member-specific sub-network (110I, 110D, 100) containing nodes connected to the identified node for L levels of nearest neighbors, wherein L is a positive integer;
   calculating a connectivity score (214) for the molecule represented by the identified node based on significance scores of each node contained in the member-specific sub-network; and
   repeating said steps of identifying a node, identifying a member-specific network and calculating a connectivity score for other nodes in the network.

2. The method of claim 1, further comprising ranking the molecules, represented by the nodes identified, for significance by ranking according to the connectivity scores (214) calculated for the nodes identified.

3. The method of claim 1 or claim 2, wherein a data set including data values characterizing molecules experimented on is provided, including significance scores (210, 308) for the molecules experimented on; and
   wherein said repeating said steps comprises repeating said steps for each node representing a molecule **characterized by** said data set.

4. The method of claim 1 or claim 2, wherein a data set including data values characterizing molecules experimented on is provided, including significance scores (210, 308) for the molecules experimented on; and
   wherein said repeating said steps comprises repeating said steps for each node included in the network.

5. The method of any of claims 1-4, wherein a data set including data values characterizing molecules experimented on is provided, including significance scores (210, 308) for the molecules experimented on; said method further comprising the step of extracting a data sub-network (110D) from the biological network provided, wherein said data sub-network contains only nodes representing molecules **characterized in** said data set, and wherein said identifying steps are carried out with regard to said data sub-network.

6. The method of claim 5, wherein said repeating said steps comprises repeating said steps for each node included in the data sub- network 9110D).

7. The method of claim 5, wherein an interesting list of molecules is provided as a subset of the molecules from the dataset, the interesting list including significance scores for the molecules in the list; and wherein said repeating said steps comprises repeating said steps for each node included in the data sub- network.

8. The method of claim 5, wherein an interesting list of molecules is provided as a subset of the molecules from the dataset, the interesting list including significance scores for the molecules in the list; and wherein said repeating said steps comprises repeating said steps only for nodes in the data sub- network (110D) that are representative of molecules in the interesting list.

9. The method of any of claims 1-8, wherein a data set including data values characterizing molecules experimented on is provided, including significance scores for the molecules experimented on, and an interesting list of molecules is provided as a subset of the molecules from the dataset, the interesting list including significance scores for the molecules in the list; said method further comprising the step of extracting an interesting sub-network (110I), wherein said interesting sub-network contains only nodes representing molecules contained in the interesting list, and wherein said identifying steps are carried out with regard to said interesting sub-network.

10. The method of any of claims 1-9, further comprising extracting at least one of said member-specific networks identified.

11. The method of any of claims 1-10, further comprising filtering nodes in the biological diagram to eliminate from consideration nodes that have been assigned a significance score that does not exceed a predefined threshold value.

12. The method of any of claims 1-11, further comprising extracting at least two of said member-specific sub-networks (110I, 110D) and combining said at least two member-specific sub-networks into a super-network (400).

**13.** The method of any of claims 2-12, further comprising selecting a subset of the ranked molecules, based on those molecules ranked relatively highest, extracting said member-specific sub-networks corresponding to the molecules in said subset, and combining said extracted member-specific sub-networks into a super-network (400).

**14.** A network-based method of identifying significant molecules, for which at least one biological network (100) is provided to include significant molecules to be identified, a data set including data values characterizing molecules experimented on is provided, and an interesting list of molecules is provided as a subset of the molecules from the dataset, the interesting list including significance scores (210, 308) for the molecules in the list, said method comprising the steps of:

identifying a node in the network;
identifying a member-specific sub-network containing nodes connected to the identified node for L levels of nearest neighbors, wherein L is a positive integer;
extracting the member-specific sub-network from the network; and
repeating said steps of identifying a node, identifying a member-specific network and extracting the member-specific sub-network from the network for each of the other nodes in the network that corresponds to a molecule in the interesting list.

**15.** The method of claim 14, further comprising combining said member specific sub-networks to form a super-network (400).

**16.** The method of claim 14 or 15, further comprising calculating a connectivity score for each molecule for which a member-specific sub-network was extracted, based on significance scores of each molecule represented by each node contained in the member-specific sub-network.

**17.** The method of claim 16, further comprising ranking the molecules, represented by the nodes identified, for significance by ranking according to the connectivity scores calculated for the nodes identified.

**18.** The method of claim 17, further comprising selecting a subset of the ranked molecules, based on those molecules ranked relatively highest, and combining said extracted member-specific sub-networks corresponding to the molecules in said subset into a super-network (400).

**19.** The method of any of claims 2-13, 17 and 18, further comprising identifying at least one nexus member based on the ranked connectivity scores.

**20.** The method of claim 1, 14 or 15, further comprising identifying a nexus member by identifying the highest connectivity score calculated.

**21.** A system for network-based identification of significant molecules, for which at least one biological network is provided to include significant molecules to be identified, comprising:

means for identifying a node in the network;
means for identifying a member-specific sub-network containing nodes connected to the identified node for L levels of nearest neighbors, wherein L is a positive integer; and
means for calculating a connectivity score for the molecule represented by the identified node based on significance scores of each node contained in the member-specific sub-network.

**22.** A computer program comprising computer program code means for performing all of the steps of any of claims 1 to 20 when said program is run on a computer.

FIG. 1

FIG. 2

FIG. 3

| Symbol | Aliases | Gene Name | # Nodes | dScore | cum DScore | Avg. d-score | d>1 | >4 nodes | sig gene? | Nodes |
|---|---|---|---|---|---|---|---|---|---|---|
| crsp2 | crsp2, crsp150, cxorf4, drip150, trap170, rgr1, exlm1, csrp | cofactor required for Sp1 transcriptional activation, subunit 2, 150kDa | 5 | 2.5674639 | 10.993886 | 2.1987772 | TRUE | TRUE | TRUE | surb7, crsp2, ercc3, med6, crsp9 |
| crsp9 | crsp9, crsp33, mgc12284, med7 | cofactor required for Sp1 transcriptional activation, subunit 9, 33kDa | 5 | 3.1198924 | 10.993886 | 2.1987772 | TRUE | TRUE | TRUE | med6, ercc3, crsp2, surb7, crsp9 |
| med6 | med6, ny-ren-28 | mediator of RNA polymerase II transcription, subunit 6 homolog (yeast) | 5 | 2.841303 | 10.993886 | 2.1987772 | TRUE | TRUE | TRUE | surb7, med6, crsp9, crsp2, ercc3 |
| rnp24 | rnp24, p24a | coated vesicle membrane protein | 5 | 2.8627603 | 10.61416 | 2.122832 | TRUE | TRUE | TRUE | rnp24, c15orf22, cdk5r1, cd9, grasp |
| insr | insr | insulin receptor | 6 | 2.1652462 | 12.145206 | 2.024201 | TRUE | TRUE | TRUE | ais, insr, lim2, igf1r, ret, igf1 |
| casp9 | casp9, ice-lap6, apaf3, mch6, apaf-3 | caspase 9, apoptosis-related cysteine protease | 6 | 2.4307728 | 10.998243 | 1.8330405 | TRUE | TRUE | TRUE | casp8, casp9, apaf1, map3k5, mycn, tnfsf10 |
| calcr | calcr, crt, ctr, ctr1 | calcitonin receptor | 5 | -2.8893747 | 8.141281 | 1.6282562 | TRUE | TRUE | TRUE | rbl1, app, calcr, bcr, grp58 |
| xab1 | xab1, ntpbp, mbdin | XPA binding protein 1 | 5 | 1.9209237 | 8.030909 | 1.6061818 | TRUE | TRUE | TRUE | lgals4, mbd2, ercc8, xpa, xab1 |
| casp10 | casp10, flice2, mch4, alps2 | caspase 10, apoptosis-related cysteine protease | 6 | 0 | 9.500938 | 1.583489667 | TRUE | TRUE | FALSE | fadd, casp8, ppp1r7, bard1, cflar, casp10 |
| sfrs6 | sfrs6, b52, mgc5045, srp55 | splicing factor, arginine/serine-rich 6 | 5 | 0 | 7.7926216 | 1.55852432 | TRUE | TRUE | FALSE | sfrs6, sfrs2, sfrs1, sfrs7, arsf |
| fh | fh, lrcc, hlrcc, fumarase, mcl, mcul1 | fumarate hydratase | 5 | 2.7912369 | 7.5593805 | 1.5118761 | TRUE | TRUE | TRUE | il6st, phgdh, mdm2, fh, atm |
| igf2 | igf2 | insulin-like growth factor 2 (somatomedin A) | 12 | 0 | 17.917358 | 1.493113167 | TRUE | TRUE | FALSE | il6, cd9, smad1, ins, atp7a, igf2r, ctcf, igf2, igfbp3, ifng, fgf8, igf1 |
| h2afx | h2afx, h2a/x, h2a.x, h2ax | H2A histone family, member X | 6 | 2.8207538 | 8.785137 | 1.4641895 | TRUE | TRUE | TRUE | brca1, h2afx, mre11a, mcl1, atm, hist2h2aa |

FIG. 4

| Symbol | Gene Name | # Nodes | dScore | cum DScore | Nodes |
|---|---|---|---|---|---|
| il-6 | interleukin 6 (interferon, beta 2) | 20 | -2.3369873 | 52.466904 | gp130, gfap, pml, c-src, cntf, mtrr, tgif, fbl-3, cia, smc, jakl, statl, cd28, ssa2, hsp-70, il-6, lif, hsp90, igfl, il-2ra |
| lif | leukemia inhibitory factor (cholinergic differentiation factor) | 18 | -2.324136 | 51.111683 | epo, statl, igfl, nmyc, gfap, p42, rpe, jakl, cntf, asp, il-6, lif, tgif, cd9, il-1ra, cav2, gp130, drg |
| c-src | v-src sarcoma (Schmidt-Ruppin A-2) viral oncogene homolog (avian) | 16 | -2.353525 | 42.231064 | hsp90, pi-plc, c-src, fak, rackl, syk, kidins220, tom111, mcp, weel, fbp2, pld1, pld2, il-6, il-1ra, cd84 |
| tgif | interleukin 10 | 14 | -2.105353 | 35.38436 | il-6, il-1ra, tnf-r, ccl1, jakl, tgif, lif, il-10r, il-10rl, rag2, c48, slam, cia, mcr |
| igfl | insulin-like growth factor 1 (somatomedin C) | 11 | -5.3893185 | 29.967812 | il-6, raftl, epo, igfbp4, als, lif, igfl, mpp2, sgkl, smc, mef2d |
| il-1ra | interleukin 1 receptor, type I | 9 | -3.4864583 | 22.509275 | mmp-13, ocif, lif, tgif, tnf-r, p85, cia, il-1ra, c-src |
| gp130 | interleukin 6 signal transducer (gp130, oncostatin M receptor) | 7 | -2.9109614 | 18.13827 | jakl, statl, il-6, cntf, lif, gp130, smc |
| epo | erythropoietin | 6 | -2.2353687 | 17.772518 | igfl, gp55, lif, epo, abpa, gfap |
| statl | signal transducer and activator of transcription 1, 91kDa | 7 | 2.0747035 | 17.58118 | usp18, jakl, il-6, gp130, statl, lif, ddb-1 |
| hsp-70 | heat shock 70kDa protein 4 | 8 | 1.9571844 | 17.312822 | tpr, hsc20, hsp90, sralpha, hsp105a, ssa2, hsp-70, il-6 |
| cd9 | CD9 antigen (p24) | 5 | 4.7494426 | 16.173779 | il-2ra, cd9, grasp, p24a, lif |
| atc | ataxia telangiectasia mutated (includes complementation groups A, C and D) | 7 | 1.8571825 | 15.789556 | dna-pk, fev, rpa2, raftl, ate, h2afx, mrel1 dna-pk, fev, rpa2, raftl, ate, h2afx, mrel1 |

FIG. 5

EP 1 727 066 A2

**FIG. 6**

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 10641492 A
- US 68204805 P
- WO 10642376 A
- WO 10784523 A
- WO 10154529 A
- WO 10928494 A

**Non-patent literature cited in the description**

- **TUSHER et al.** Significance analysis of microarrays applied to the ionizing radiation response. *PNAS,* 24 April 2001, vol. 98 (9), 5116-5121
- **LARKIN et al.** Cardica transcriptional response to acute and chronic angiotensin II treatments. *Physiol Genomics,* 2004, vol. 18, 152-166
- **LOPES et al.** Pathophysiology of plaque instability: insights at the genomic level. *Prog Cardi ovasc Dis,* 2002, vol. 44, 323-328
- **JOHNSON et al.** Genomic profiles and predictive biological networks in oxidant-induced atherogenesis. *Physiol Genomics,* 2003, vol. 13, 263-275